# EUROPEAN PATENT SPECIFICATION

(11) **EP 2 617 303 B1**
(45) Date of publication and mention of the grant of the patent: **16.12.2015**
(21) Application number: 11873529.9
(22) Date of filing: 19.10.2011
(51) Int. Cl.: A24F 47/00

(54) **REPLACEABLE GENERAL ATOMIZING HEAD**
AUSTAUSCHBARER ALLGEMEINER SPRÜHKOPF
TÊTE DE PULVÉRISATION GÉNÉRALE REMPLAÇABLE

(30) Priority: 28.09.2011 CN 201110302179
(43) Date of publication of application: 24.07.2013
(73) Proprietor: Joyetech (Changzhou) Electronics Co., Ltd., Jiangsu 213022 (CN)
(72) Inventor: QIU, Weihua, Changzhou Jiangsu 213022 (CN)
(74) Representative: Boult Wade Tennant
(86) International application number: PCT/CN2011/080979
(87) International publication number: WO 2013/044538

(56) References cited:
- WO-A1-2009/155734
- CN-A- 101 986 906
- CN-U- 201 781 984
- CN-U- 201 781 984
- CN-U- 201 830 900
- CN-U- 201 900 065
- CN-Y- 201 072 979

## Description

### TECHNICAL FIELD

The present invention relates to an electronic cigarette, in particular to a replaceable universal atomizing head.

### BACKGROUND TECHNOLOGY

The electronic cigarette, now mainly used in some developed countries in Europe and the United States, primarily used to replace traditional cigarettes. With the continuous improvement of living standards in China, people are also constantly pursuing a higher quality of life, and gradually realize the serious harm of smoking; therefore, quitting smoking gradually becomes a common understanding. Therefore, the alternatives such as the electronic cigarette are gradually welcomed by people.

Electronic cigarettes have the same look of cigarettes, and have a similar taste or even better taste than general cigarette tastes. Electronic cigarettes, also like cigarettes, can inhale the smoke, suck out the taste and feeling. Electronic cigarettes are mainly used to give up smoking and to replace cigarettes. Electronic cigarette is a non-burning alternative product, is similar to some of the characteristics of general cigarettes, can be refreshing, and can meet smokers pleasure and the using habit of many years. But it essentially differs from the general cigarettes, because the electronic cigarette does not burn, has no tar, and does not have more than 460 kinds of chemical substances which cause respiratory and cardiovascular diseases produced by burning of smoke, thereby removing the ordinary smoke carcinogens. Electronic cigarettes will not cause passive smoking hazards or environmental pollution.

The atomizer and the battery rod are two major components of the electronic cigarette. The battery rod consists of a PCB board, a rechargeable battery, and various other circuits; the atomizer consists of an atomizing device and a cartridge. The Application No. CN201878765U of a Utility Model Patent discloses an atomizer (see Figure 10), wherein the atomization device of the atomizer includes a main body 330 of the atomizer, a suction nozzle seat 310, a heating device 311 and a liquid guiding component, wherein one end of the suction nozzle seat provides an atomizing chamber 313, the other end of the suction nozzle seat has a vent hole 314 which communicate with an atomizing chamber, wherein said heat means is fixed in the atomizing chamber; the liquid guiding component includes a suction nozzle and a liquid guiding rope 320, wherein one end of the suction nozzle is inserted into the liquid storage cavity of a liquid storage box, wherein the contacting parts form a liquid seal; the other end of the suction nozzle connects with an atomizing chamber of the suction nozzle seat, wherein said liquid guide rope is wound on the heat means 311; both ends of the liquid guiding rope are introduced into the suction nozzle.

A connecting assembly of the atomizing device 30 is fixed to the other end of the housing 10; the connecting assembly includes a main body 330 of the atomizer, a contacting conductor 331 connected to the heating device 311 by conductors, and a contacting conductor base 332. One end of the main body 330 of the atomizer locating in the housing forms a cavity for receiving and holding the atomizing assembly, the sleeve 312, and the other atomizing assemblies are fixed in said cavity, wherein the sleeve 312 cooperates with the cavity in over surplus manner. The sidewall of the main body of the atomizer is provided with an inlet hole 333 for conducting air which communicates with the cavity. The contacting conductor base 332 is fixed on the other end of the main body of the atomizer exposed the housing 10, wherein two contacting conductor 331 are fixed in the contacting conductor base 332, connecting the power supply positive and negative electrodes respectively, wherein each contacting conductor has a hole for contacting thimble accessing. Further, the outer surface of the main body 330 of the atomizer has a step, the main body 330 of the atomizer for supporting the atomizer assembly firstly, and for connecting the power supply apparatus secondly.

The atomizing apparatus of the above-described structures can atomize the liquid smoke, but also has the following disadvantages:
Firstly, the configuration of the atomizing device is a heating device, heated by a battery-powered and making the surrounding electronic cigarette juice volatilized to form the smog, which makes the suction time to reach the effects of the "puff". Traditional electronic cigarette atomizer with the housing is a unibody, and the atomization device can be used just a few days and cannot be replaced after damage, and the use cost is high. If you have been using a separate atomizing device for a long time, the taste is very poor, and the amount of smoke is also very small;
Secondly, the two contacting conductors 331 are a conductive positive electrode and a negative electrode, respectively; the electrically positive and negative electrodes and the both ends of the heat means 311 are welded by the wire; Because the outer diameter of the main body 330 of the atomizer is only 10mm and the assembly of these parts is by hand, it is a high difficulty and increases the labor intensity;
Thirdly, since the concentration of the liquid smoke itself is relatively high, while in the suction nozzle is provided a liquid guiding rope, which occupies the space of the suction nozzle, resulting in the liquid smoke flowing downward slowly. On the other hand, when the liquid smoke is flowing, the heat means 311 has been working, due to the flow seed of the liquid smoke is slower than the operating speed of the heating apparatus, i.e., only a small portion of the liquid smoke can reach the heating device, it does not meet the normal proportion of required liquid smoke, the gas in the mouth is a dry taste, this phenomenon is called dry combustion.

Similar designs of atomizing apparatus are disclosed in CN 201 781 984 U, CN 201 900 065 and CN 201 830 900 U.

### SUMMARY OF THE INVENTION

One of the purposes of the present invention is to provide a replaceable universal atomizing head that has a simple structure which is easy to assemble and can prevent dry combustion.

The technical solution to achieve the object of the present invention is provided by claim 1.

Generally, a replaceable universal atomizing head includes a support base with an atomizing chamber, a heating device, and a fluid guide member, the heating device is fixed in the atomizing chamber of the support base, and said liquid guiding member comprises a liquid guiding rope, a liquid guiding nozzle and a liquid guiding nozzle seat connected integrally with the liquid guiding nozzle, wherein one end of the liquid guide rope pierced from the liquid guiding nozzle seat and reaches heating device, characterized in that: further comprising a conductive ring, said support base is fixed in the middle of the conductive ring cavity, pressing a wire connected to one end of the heating device between the conductive ring and the support base; the wall surface of the inner hole of the liquid guiding nozzle and the liquid guiding nozzle seat provides a liquid guiding slot; one end of the liquid guiding nozzle seat is inserted into one end of the conductive ring cavity; the other end of the conductive ring cavity provides a conductive member, which comprises a conductor and insulator, the conductor is fixed on the insulator and welded with wire connected to the other end of the heating device, wherein the insulator with vent holes is fastened in the conductive ring cavity.

Using the above scheme, said support base is fixed in the middle of the conductive ring cavity, pressing a wire connected to one end of the heating device between the conductive ring and the support base, in such way without welding the conductive anode and the heating device artificially, which can reduce process and labor intensity. A conductive member is fitted in the other end of the conductive ring cavity, so almost all parts of the universal atomizing head are mounted in the conductive ring, and the entire structure of the universal atomizing head becomes simple and compact. Combined with the practice of the workers, making a universal atomizing head relative to the existing atomizing head can save 2-3 minutes, and the cost is three-fourths of that of the existing atomizing head, and so whether for the production time, labor intensity or cost, the universal atomizing head of the present invention has a great advantage. The wall surface of the inner hole of the liquid guiding nozzle and the liquid guiding nozzle seat provides a liquid guiding slot, which can improve the flow rate of the liquid smoke by draining the liquid smoke, when heating, it realizes the liquid smoke provided and the heating device with an appropriate so that avoids the phenomenon of dry combustion.

Another object of the present invention is to provide an atomizer, which can be replaced with the universal atomizing head after damage from the long-time use of the electronic cigarette, and is beneficial to reducing the use costs.

Technical solutions to achieve the purposes of this invention are as follows:
An atomizer is provided with the replaceable universal atomizing head according to claim 1, further comprising an atomization assembly, an outer threaded sleeve, said atomization assembly provides a universal receiving cavity for accommodating the universal atomizing head, which is detachably loaded in the receiving cavity, said connecting outer threaded sleeve and the atomization assembly contact the conductive rings and the conductors of the universal atomizing head.

Using the above scheme, said atomization assembly provides a universal receiving cavity for accommodating the universal atomizing head, which is detachably loaded in the receiving cavity; if the universal atomizing head loaded in the receiving cavity is inefficient or unusable, you can remove the universal atomizing head anytime, anywhere, from the receiving cavity, without replacing the atomizer overall, which helps to reduce the use cost.

Another object of the present invention is to provide an electronic cigarette, whose universal atomizing head can be replaced at any time, which can reduce the production cost.

Technical solutions to achieve the purposes of this invention are as follows:
An electronic cigarette is provided with the atomizer as claimed in claim 5, wherein the electronic cigarette further comprises a battery rod, the battery rod and the outer threaded sleeve of the atomizer threaded connection.

The atomizer of the electronic cigarette of the present invention is the atomizer of the aforementioned scheme; therefore, both the advantages of atomizer as well as universal atomizing head mounted on the atomizer are provided.

Now, combining the drawings and specific embodiments to fully describe the structures and advantages of the present invention.

### BRIEF DESCRIPTION OF THE DRAWINGS

Figure 1 is a cross-sectional structure diagram of replaceable universal atomizing head of the present invention;
Figure 2 is a perspective view of the common atomizing head;
Figure 3 is the cross-sectional configuration diagram of the universal atomizing head, which is based on Figure 1, adding a liquid absorbing member;
Figure 4 is the split structure diagram of the atomizer of the present invention;
Figure 5 is the sectional structural diagram of the invention of the first embodiment of the atomizer;
Figure 6 is the sectional structural diagram of the invention of the second embodiment of the atomizer;
Figure 7 is the sectional structural view of the invention of the third embodiment of the atomizer;
Figure 8 is the sectional structural diagram of the present invention of the fourth embodiment of the atomizer;
Figure 9 is the sectional structural diagram of the electronic cigarette of the present invention; and

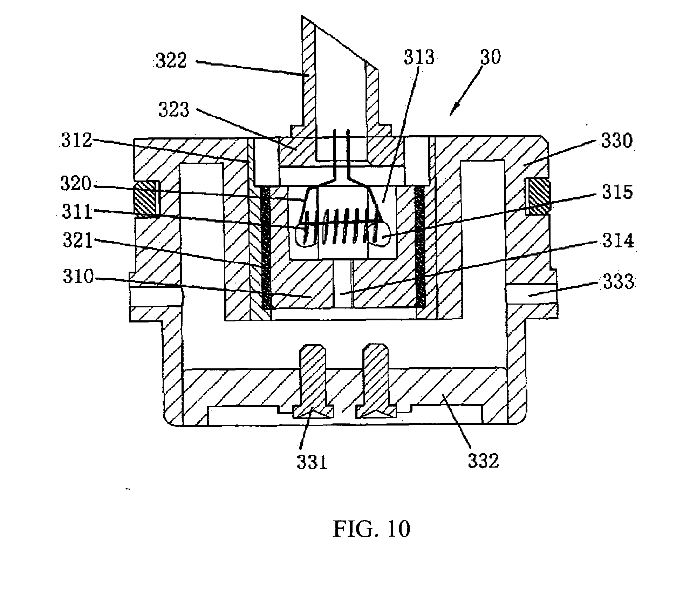

Figure 10 is the schematic structural view of a prior art atomizer.

In the drawings, 10 is a universal atomizing head, 100 is a support base, 101 is a heating device, 102 is a conductive ring, 103 is an atomizing chamber, 104 is a vent hole, 105 is a liquid absorbent member, 106 is a liquid guiding rope, 107 is a liquid guiding nozzle, 108 is a liquid guiding nozzle seat, 109 is a liquid guiding slot, 110 is a conductor, 111 is an insulator; 20 is an atomizer, 200 is a cartridge, 201 is a housing, 202 is a first sleeve, 203 is a small aperture segment, 204 is a large aperture segment, 2040 is an annular retaining boss, 205 is an insulation sleeve, 206 is a sleeve, 207 is a conductive negative electrode, 208 is a conductive positive electrode, 209 is an insulating member, 210 is a notch, 211 is a screw, 212 is a circlip; 30 is an electronic cigarette, 300 is a battery rod.

### DETAILED DISCLOSURE OF THE INVENTION

Now, the present invention will be further described combining with the drawings and specific embodiments.

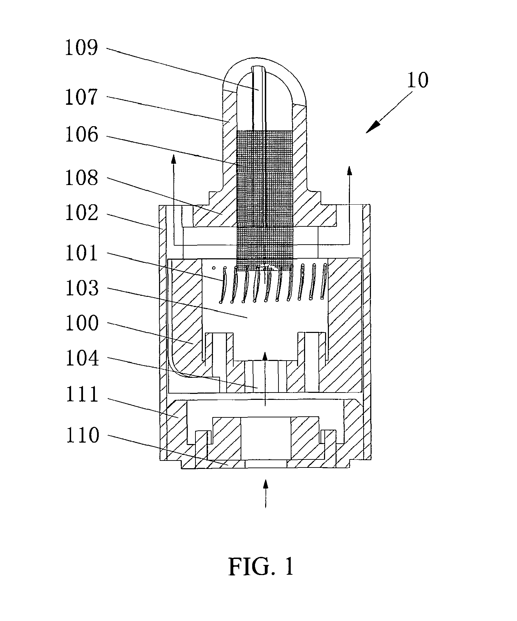

Referring to Figures 1 to 3, a universal atomizing head 10 of the present invention, which comprises a support base 100 with an atomizing chamber, a heating device 101, a conductive ring 102, and a liquid guiding member. The support base 100 is made of ceramic, in order to install the heating device 101, an atomizing chamber 103 is provided in the support base 100. In order to permit the gas to enter into the atomization chamber 103, a vent hole 104 is provided in the bottom of the support base 100. The heating device 101 is a heating wire made of the metal, fixed to the atomizing chamber 103 of the support base 100; both ends of the heating device before assembled into a universal atomizing head are welded with the wire. On one hand, the conductive ring 102 is a conductive negative of the universal atomizing head; on the other hand is used to mount other components.

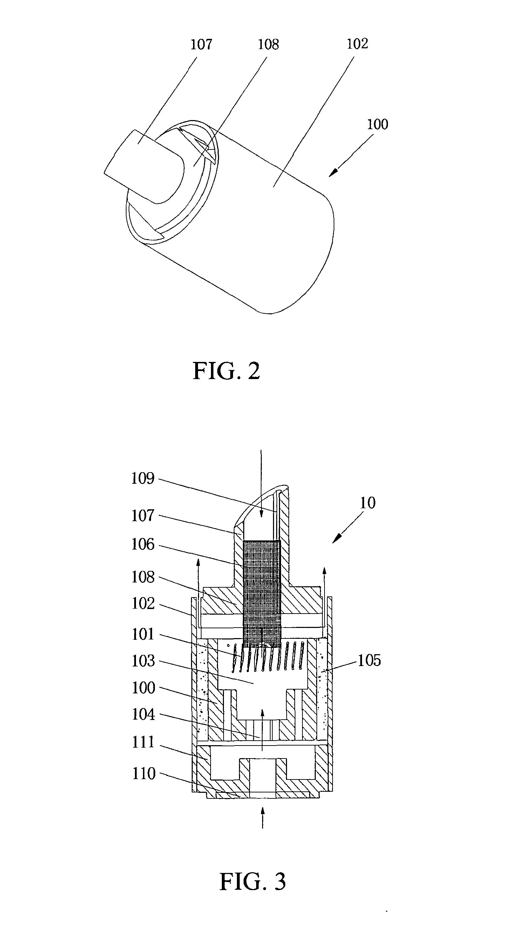

Referring to Figure 1, said support base 100 is fixed in the middle of the conductive ring 102 cavity, pressing a wire connected to one end of the heating device between the conductive ring and the support base, making the conductor and the conductive ring maintain good contact, so that, while using this way, without welding the conductive anode and the heating device artificially, which can reduce process and labor intensity. Referring to Figure 3, in order to be able to reduce adverse effects on the universal atomizing head produced by a little atomized liquid smoke refluxed to the universal atomizing head, a layer of a liquid absorbing member 105 is wrapped in the outer surface of the support base 100, which is a nickel mesh. Wrapped in the liquid absorbing member, the support base 100 is mounted to the middle of the cavity of the conductive ring 102, also pressing a wire connected to one end of the heating device between the conductive ring and the nickel mesh, making the conductor and the conductive ring maintain good contact.

Referring to Figures 1 to 3, said liquid guiding member comprises a liquid guiding rope 106, a liquid guiding nozzle 107 and a liquid guiding nozzle seat 108 connected integrally with the liquid guiding nozzle. One end of the liquid guide rope pierced from the liquid guiding nozzle seat and reaches heating device; in order to improve the heating efficiency of the heating device to the liquid smoke, the liquid guiding rope is preferably wound on the upper of the heating device 101 (from the figure), the flow path of the liquid smoke is almost in the whole heating device; therefore, arranged in the such manner, the heating effect of heating device 101 can improve the liquid smoke heating efficiency. Although the liquid guiding rope 106 helps the uniformity of the liquid smoke flowed to the heating device, the liquid smoke flows slowly because of the liquid guiding rope, so the wall surface of the inner hole of the liquid guiding nozzle 107 and the liquid guiding nozzle seat 108 provides a liquid guiding slot 109, draining the liquid smoke by the liquid guiding slot to improve the flow rate of the liquid smoke, when heating, it realizes the liquid smoke provided and the heating device with an appropriate so that avoids the phenomenon of dry combustion. Liquid guiding nozzle 107 and the liquid guiding nozzle seat 108 is integrally molded, so the liquid guiding slot 109 which on the liquid guiding nozzle 107 and the nozzle seat 108 is a communicating slot. The number of the liquid guiding slot 109 relates to the arrangement of the heating device; when the number of the liquid guiding slot 109 of both said liquid guiding nozzle and the liquid guiding nozzle seat is one, the heating device 101 is fitted in the atomizing chamber of the support base, and its ends are in the same horizontal line - the reason is that: when the heating device 101 is in the same horizontal line, the flow path of liquid smoke along the surface of the heating device is short, therefore, when heated atomizing speed is fast, if the liquid smoke is excessive, there will be leakage; therefore, one liquid guiding slot can meet the requirements, and a universal atomizing head provided one liquid guiding slot is suitable for the persons who use a small amount of electronic cigarette. If the number of the liquid guiding slot of both said liquid guiding nozzle and the liquid guiding nozzle seat is two or three, the heating device 101 is disposed in the atomizing chamber of the support base with one end high, and the other end low, i.e. the heating device in the atomizing chamber of the support base 100 is arranged inclined the reason is that: the heating device is inclined arranged in the atomizing chamber of the support base 100, prolonging the flow path of the liquid smoke, and thus does not leak; so, by increasing the supply amount of the liquid smoke by increasing the number of liquid guiding slots, and the amount of smoke is also improved. A universal atomizing head with two or more liquid guiding slots is suitable for the persons who use a large amount of electronic cigarette. Since the universal atomizing head of the present invention can be replaced, the same person can select a specific number of slots of the universal atomizing head in accordance with the demand of the amount of smoke; it is quite convenient for the user. One end of the liquid guiding nozzle seat 108 is inserted into one end of the cavity of the conductive ring 102; the liquid guiding nozzle seat 108 is a cylindrical shape; on the outer circumference of the liquid guiding nozzle seat 108 along its axial direction has a truncated sectional, so the liquid guiding nozzle seat 108 is mounted to the conductive ring 102; a channel is provided between the liquid guiding nozzle seat 108 and the conductive ring 102, which is formed for the atomized smoke of the liquid smoke to go through.

Referring to Figures 1 to 3, the other end of the cavity of the conductive ring 102 has a conductive member, wherein the conductive member includes a conductor 110 and an insulator 111. The conductor 110 is the positive electrode of the universal atomizing head; the conductor 110 is fixed on the insulator 111 and welds with wire connected to the other end of the heating device; the conductive body 110 is in a ring shape; the conductor 110 is mounted in the annular fitting groove provided on the axial end surface of the insulator. The insulator is fastened to the cavity of the conductive ring on an insulator provided with a vent hole, which for when smoking the negative pressure airflow through. The conductive member is disposed in the other end of the conductive ring cavity to increase the compactness of the structure, to reduce the use of parts, and is easy to install.

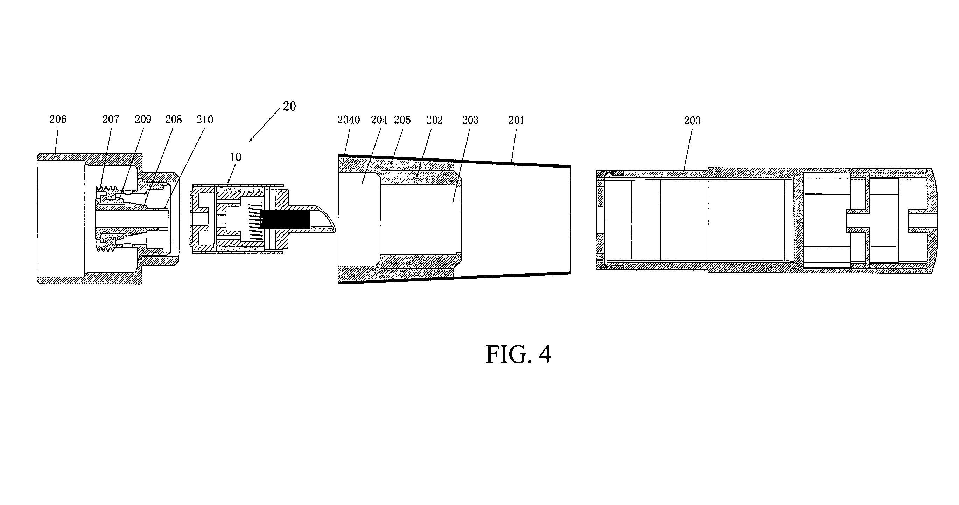

Universal atomizing head 10 of the present invention is a core of the atomizer; therefore, an atomizer structure which is produced by universal atomizing head 10 of the present invention is described below:
Referring to Figure 4, the atomizer 20 of the present invention, which comprises an atomization assembly, a universal atomizing head and an outer threaded sleeve, wherein said universal atomizing head is the Universal atomizing head 10 of the embodiment which the above Figs 1 to 3 described.

In order to facilitate the replacement of the universal atomizing head, a receiving cavity, which can accommodate the universal atomizing head, is set on the atomization assembly, and the universal atomizing head 10 is detachably loaded in the receiving cavity. The atomization assembly includes a cartridge 200, a housing 201, and a first sleeve 202. Cartridge 200 consists of a box for storing smoke fluid and a nozzle, which are connected together, can be directly plugged on the housing 201; the cartridge 200 is connected to one end of the housing 201, and is pluggable connecting with the housing, to be assembled with a plug and play manner. The first sleeve 202 is fixed to the other end of the housing 201, and has a stepped bore, whose segment 203 of the smaller aperture is a receiving cavity on the atomization assembly for accommodating the universal atomizing head 10, which is fitted in the gap in the receiving cavity, the liquid guiding nozzle is inserted to the box of the cartridge 200 after piercing the receiving cavity, the end of the positive electrode of the universal atomizing head 10 extends to a large aperture segment 204 of the stepped bore, occupying a part of the space. Corresponding to the large aperture segment 204, the outer wall of the first sleeve 202 is provided an annular retaining boss 2040, corresponding to the small aperture segment 203, the outer wall of the first sleeve 202 is sleeved an insulated sleeve 205, because of the heating device of the universal atomizing head 10 is located in the small aperture segment 203, the heat emitted to reach 60 degrees in its work, the heat will be transmitted to the outside of the housing, when smoking, hand will grip it, the insulated sleeve 205 absorbs the heat emitted by the heating device, to reduce the heat transmitted to the outer wall of the casing 201, and using without burning. The housing 201 and the first sleeve 202 are assembled in press fitting, therefore can limit the location of the insulated sleeve 205 by the annular retaining boss 2040, and the insulated sleeve 205 is not displacing or sliding in the first sleeve.

Said outer threaded sleeve includes a sleeve 206, a conductive negative electrode 207, a conductive positive electrode 208 and an insulating member 209, wherein one end of said conductive negative electrode 207 located in the sleeve is provided with an outer thread, and the conductive negative electrode provides a stepped down through hole. The conductive positive electrode 208 is located in the stepped down through hole of the conductive negative electrode 207; the end of conductive positive electrode 208 has a notch 210 for the gas passed through. The insulating member is interposed in the conductive positive electrode and the conductive negative electrode.

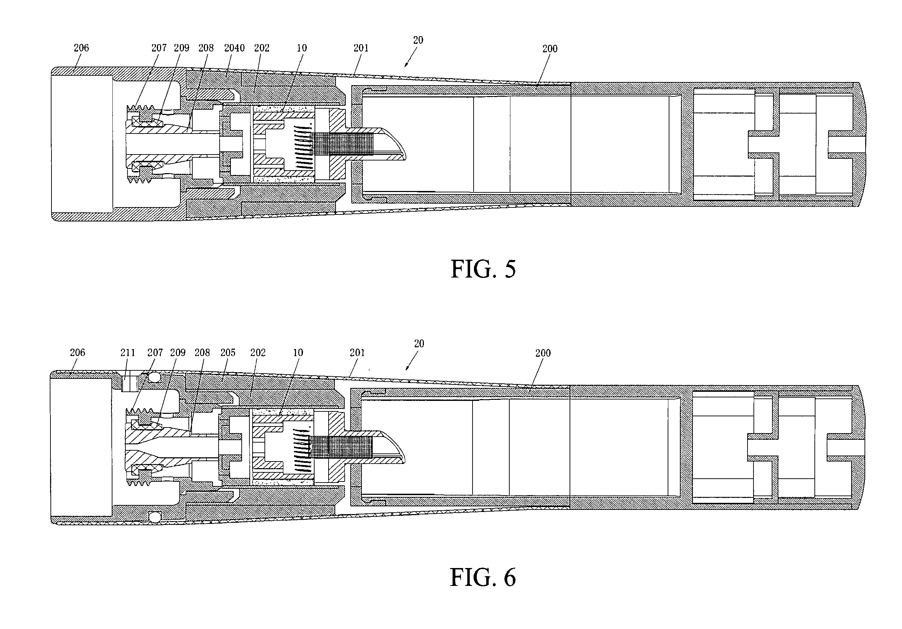

The outer threaded sleeve and atomization assembly are connected to contact with the conductive rings and the conductor of the universal atomizing head, wherein, the conductive negative electrode 207 contacts with conductive ring 102 of the universal atomizing head 10, and the conductive positive electrode 208 contacts with the conductor 110. The connections of the outer threaded sleeve and atomization assembly are the following:
Referring to Figure 5, for the first way: the atomization assembly connects with the outer threaded sleeve threadably; the internal thread is provided on the inner wall of the section 204 of the large aperture in the first sleeve of the atomization assembly; the outer end of the outer threaded sleeve is provided on the outer thread. When assembling, the universal atomizing head is inserted to the small aperture segment 203 of the first sleeve of the atomization assembly firstly, and then connected with the outer thread of the sleeve 206 with the internal thread of the large aperture section.

With reference to Figure 6, for a second way: a radial through hole is provided in the circumferential surface of the housing 201 of the atomization assembly; a threaded bore is provided on the sleeve 206 of the outer threaded sleeve; when assembling, the universal atomizing head is inserted to the small aperture segment 203 of the first sleeve of the atomization assembly firstly, then the housing 201 of the atomization assemblysets in the sleeve 206, at last fastening the sleeve 206 and the housing 201 with a screw 211.

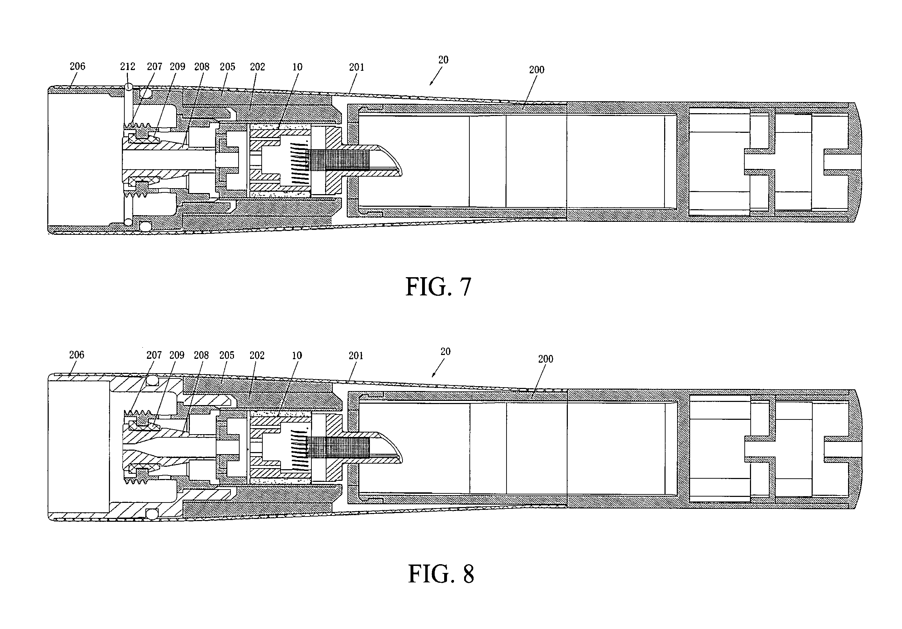

Referring to Figure 7, for a third way: a radial through hole is provided in the circumferential surface of the housing 201 of the atomization assembly; a circlip 212 is provided on the sleeve 206 of the out threaded sleeve; when assembling, the universal atomizing head is inserted to the small aperture segment 203 of the first sleeve of the atomization assembly firstly, then the housing 201 of the atomization assembly sets in the sleeve 206, a bump on the circlip 212 pops into the through hole in the housing 201, locking the housing 201 and the sleeve 206.

Referring to Figure 8, for a fourth way: the rod housing 201 of the atomization assembly directly sets in the sleeve 206 of the outer threaded sleeve to form a plug connection.

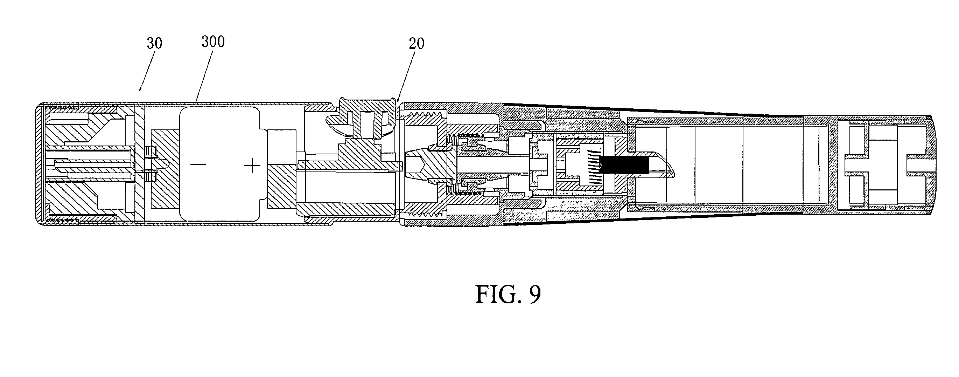

Referring to Figure 9, the electronic cigarette of the present invention 30 consists of the atomizer and battery rod 300, wherein the atomizer is the atomizer 20 of the representation embodiments of figures 4 to 8; the end of the battery rod comprises internal threads; battery rod 300 connects the outer thread of the conductive negative electrode 207 which is mounted in the sleeve 206 threadably by the external of the inner thread of end, thereby forming the electronic cigarette of the present invention.

## Claims

1. A replaceable universal atomizing head (10), comprising a support base (100) with an atomizing chamber (103), a heating device (101), and a fluid guide member, wherein the heating device is fixed in the atomizing chamber of the support base, and wherein said liquid guiding member comprises a liquid guiding rope (106), a liquid guiding nozzle (107), and a liquid guiding nozzle seat (108) connected integrally with the liquid guiding nozzle, wherein one end of the liquid guide rope is pierced from the liquid guiding nozzle seat and reaches the heating device, further comprising a conductive ring (102), wherein said support base is fixed in the middle of the conductive ring cavity, wherein one end of the liquid guiding nozzle seat is inserted into one end of the conductive ring cavity; wherein the other end of conductive ring cavity provides a conductive member, which comprises a conductor (110) and insulator (111), wherein the conductor is fixed on the insulator and welds with wire connected to the other end of the heating device,
**characterized in that**:
said support base is fixed in the middle of the conductive ring cavity thereby pressing a wire connected to one end of the heating device between the conductive ring and the support base;
wherein the wall surface of the inner hole of the liquid guiding nozzle and the liquid guiding nozzle seat provides a liquid guiding slot (109) and;
wherein the insulator with vent holes (104) is fastened in the conductive ring cavity.

2. A replaceable universal atomizing head according to claim 1, **characterized in that**:
said liquid guiding nozzle comprises one liquid guiding slot, and said liquid guiding nozzle seat comprises one liquid guiding slot, and wherein the heating device is fitted in the atomizing chamber of the support base, its both ends being in the same horizontal line.

3. A replaceable universal atomizing head according to claim 1, **characterized in that**:
said liquid guiding nozzle comprises two or three liquid guiding slots, and said liquid guiding nozzle seat comprises two or three liquid guiding slots, wherein the heating device is situated in the atomizing chamber of the support base with one end high the other end low.

4. A replaceable universal atomizing head according to any of claims 1 to 3, **characterized in that**:
a liquid absorbing member (105) is provided between the support base and the conductive ring.

5. An atomizer comprising the replaceable universal atomizing head according to claim 1, wherein further comprising an atomization assembly comprising a sleeve provided with an outer thread, **characterized in that**:
said atomization assembly provides a universal receiving cavity for accommodating the universal atomizing head, which is detachably loaded in the receiving cavity, wherein said outer threaded sleeve, connected to the atomization assembly, is in contact with the conductive rings and the conductors of the universal atomizing head.

6. An atomizer according to claim 5, **characterized in that**:
said atomization assembly comprises a cartridge (200), a housing (201), and a first sleeve (202), wherein the cartridge is connected to one end of the housing, wherein the first sleeve is fixed to the other end of the housing, wherein the first sleeve has a stepped bore, wherein the smaller aperture section (203) of the stepped bore is a receiving cavity on the atomization assembly for accommodating universal atomizing head.

7. An atomizer according to claim 6, **characterized in that**:
an annular retaining projection on the first sleeve is set corresponding to the outer wall of the segment (204) of the large aperture, and an insulation jacket sleeved on the first sleeve is set corresponding to the outer wall of the segment of the small aperture.

8. An atomizer according to claim 5, **characterized in that**:
said outer threaded sleeve comprises a sleeve (206), a conductive negative electrode (207), a conductive positive electrode (208) and an insulating member (209), wherein one end of said conductive negative electrode located in the sleeve comprises an outer thread, the conductive negative electrode comprises a stepped down through a hole, in which the conductive positive electrode is located, wherein the insulating member is interposed in the conductive positive electrode and the conductive negative electrode.

9. An atomizer according to any of claims 5 to 8, **characterized in that**:
said atomization assembly and the outer threaded sleeve are connected threadably; or the atomization assembly covers the outer threaded sleeve by screw fastening;
or the atomizing rod covers the outer threaded sleeve secured by circlip; or atomization assembly covers the outer threaded sleeve to form a plug connection.

10. An electronic cigarette comprising the atomizer according to claim 5, wherein the electronic cigarette further comprises a battery rod (300), **characterized in that**: the battery rod is threadably connected to the outer threaded sleeve of the atomizer.

## Patentansprüche

1. Auswechselbarer universeller Zerstäuberkopf (10), der einen Stützfuß (100) mit einer Zerstäuberkammer (103), eine Heizvorrichtung (101) und ein Fluidführungselement aufweist, wobei die Heizvorrichtung in der Zerstäuberkammer des Stützfußes befestigt ist und wobei das Flüssigkeitsführungselement ein Flüssigkeitsführungsseil (106), eine Flüssigkeitsführungsdüse (107) und einen Flüssigkeitsführungsdüsensitz (108) aufweist, der einstückig mit der Flüssigkeitsführungsdüse ausgebildet ist, wobei ein Ende des Flüssigkeitsführungsseils von dem Flüssigkeitsführungsdüsensitz durchdrungen wird und bis zur Heizvorrichtung reicht, und der ferner einen leitfähigen Ring (102) aufweist, wobei der Stützfuß in der Mitte des Hohlraums des leitfähigen Rings befestigt ist, wobei ein Ende des Flüssigkeitsführungsdüsensitzes in ein Ende des Hohlraums des leitfähigen Rings eingesetzt ist, wobei das andere Ende des Hohlraums des leitfähigen Rings ein leitfähiges Element bereitstellt, das einen Leiter (110) und einen Isolator (111) umfasst, wobei der Leiter an dem Isolator befestigt und mit dem Draht verschweißt ist, der mit dem anderen Ende der Heizvorrichtung verbunden ist,
**dadurch gekennzeichnet, dass**
der Stützfuß in der Mitte des Hohlraums des leitfähigen Rings befestigt ist und dadurch einen Draht, der mit einem Ende der Heizvorrichtung verbunden ist, zwischen den leitfähigen Ring und den Stützfuß drückt;
wobei die Wandfläche des inneren Lochs der Flüssigkeitsführungsdüse und der Flüssigkeitsführungsdüsensitz eine Flüssigkeitsführungsnut (109) ausbilden; und wobei der Isolator Lüftungslöcher (104) aufweist und in dem Hohlraum des leitfähigen Rings befestigt ist.

2. Auswechselbarer universeller Zerstäuberkopf nach Anspruch 1, **dadurch gekennzeichnet, dass**:
die Flüssigkeitsführungsdüse eine Flüssigkeitsführungsnut aufweist und der Flüssigkeitsführungsdüsensitz eine Flüssigkeitsführungsnut aufweist, wobei die Heizvorrichtung so in der Zerstäuberkammer des Stützfußes montiert ist, dass sich ihre beiden Ende auf derselben horizontalen Linie befinden.

3. Auswechselbarer universeller Zerstäuberkopf nach Anspruch 1, **dadurch gekennzeichnet, dass**:
die Flüssigkeitsführungsdüse zwei oder drei Flüssigkeitsführungsnuten aufweist und der Flüssigkeitsführungsdüsensitz zwei oder drei Flüssigkeitsführungsnuten aufweist, wobei die Heizvorrichtung so in der Zerstäuberkammer des Stützfußes montiert ist, dass ein Ende höher als das andere angeordnet ist.

4. Auswechselbarer universeller Zerstäuberkopf nach einem der Ansprüche 1 bis 3, **dadurch gekennzeichnet, dass**:
zwischen dem Stützfuß und dem leitfähigen Ring ein flüssigkeitsabsorbierendes Element (105) angeordnet ist.

5. Zerstäuber, der den auswechselbaren universellen Zerstäuberkopf nach Anspruch 1 umfasst, wobei der Zerstäuber ferner eine Zerstäuberbaugruppe aufweist, die eine mit einem Außengewinde versehene Hülse aufweist, **dadurch gekennzeichnet, dass**:
die Zerstäuberbaugruppe einen universellen Aufnahmehohlraum für die Aufnahme des universellen Zerstäuberkopfes aufweist, der abnehmbar in den Aufnahmehohlraum eingebracht ist, wobei die mit einem Außengewinde versehene und mit der Zerstäuberbaugruppe verbundene Hülse mit den leitfähigen Ringen und den Leitern des universellen Zerstäuberkopfes in Kontakt ist.

6. Zerstäuber nach Anspruch 5, **dadurch gekennzeichnet, dass**:
die Zerstäuberbaugruppe eine Kartusche (200), ein Gehäuse (201) und eine erste Hülse (202) aufweist, wobei die Kartusche mit einem Ende des Gehäuses verbunden ist, die erste Hülse an dem anderen Ende des Gehäuses befestigt ist, die erste Hülse eine gestufte Bohrung aufweist und der kleinere Öffnungsabschnitt (203) der gestuften Bohrung zum Aufnehmen des universellen Zerstäuberkopfes einen Aufnahmehohlraum an der Zerstäuberbaugruppe bildet.

7. Zerstäuber nach Anspruch 6, **dadurch gekennzeichnet, dass**:
ein ringförmiger Haltevorsprung an der erste Hülse mit der Außenwand des Segments (204) der großen Öffnung in Übereinstimmung gebracht ist und ein die ersten Hülse umhüllender Isolationsmantel mit der Außenwand des Segments der kleinen Öffnung in Übereinstimmung gebracht ist.

8. Zerstäuber nach Anspruch 5, **dadurch gekennzeichnet, dass**:
die mit dem Außengewinde versehene Hülse eine Hülse (206), eine leitfähige negative Elektrode (207), eine leitfähige positive Elektrode (208) und ein Isolationselement (209) aufweist, wobei ein Ende der in der Hülse angeordneten leitfähigen negativen Elektrode ein Außengewinde aufweist, die leitfähige negative Elektrode ein stufenförmig verkleinertes Durchgangsloch aufweist, in dem sich die leitfähige positive Elektrode befindet, wobei das Isolationselement zwischen der leitfähigen positiven Elektrode und der leitfähigen negativen Elektrode angeordnet ist.

9. Zerstäuber nach einem der Ansprüche 5 bis 8, **dadurch gekennzeichnet, dass**:
die Zerstäuberbaugruppe und die mit einem Außengewinde versehene Hülse miteinander verschraubt sind;
oder die Zerstäuberbaugruppe die mit einem Außengewinde versehene Hülse durch Aufschrauben überdeckt;
oder der Zerstäuberstab die mit einem Außengewinde versehene und mit einem Sicherungsring gesicherte Hülse bedeckt;
oder die Zerstäuberbaugruppe die mit einem Außengewinde versehene Hülse bedeckt, um eine Steckverbindung auszubilden.

10. Elektronische Zigarette, die den Zerstäuber nach Anspruch 5 umfasst, wobei die elektronische Zigarette ferner einen Batteriestab (300) aufweist, **dadurch gekennzeichnet, dass** der Batteriestab mit der mit einem Außengewinde versehenen Hülse des Zerstäubers schraubbar verbunden ist.

## Revendications

1. Tête d'atomisation universelle remplaçable (10), comprenant une base de support (100) avec une chambre d'atomisation (103), un dispositif de chauffage (101), et un élément de guidage de fluide, dans laquelle le dispositif de chauffage est fixé dans la chambre d'atomisation de la base de support, et dans laquelle ledit élément de guidage de liquide comprend un cordon de guidage de liquide (106), une buse de guidage de liquide (107), et un siège de buse de guidage de liquide (108) relié d'un seul tenant avec la buse de guidage de liquide, où une extrémité du cordon de guidage de liquide est percée à partir du siège de buse de guidage de liquide et atteint le dispositif de chauffage, comprenant en outre une bague conductrice (102), où ladite base de support est fixée au milieu de la cavité de bague conductrice, où une extrémité du siège de buse de guidage de liquide est insérée dans une extrémité de la cavité de bague conductrice ; où l'autre extrémité de la cavité de bague conductrice fournit un élément conducteur, qui comprend un conducteur (110) et un isolant (111), où le conducteur est fixé sur l'isolant et est soudé à un fil relié à l'autre extrémité du dispositif de chauffage,
**caractérisée en ce que** :
ladite base de support est fixée au milieu de la cavité de bague conductrice pressant ainsi un fil relié à une extrémité du dispositif de chauffage entre la bague conductrice et la base de support ;
dans laquelle la surface de paroi du trou intérieur de la buse de guidage de liquide et du siège de buse de guidage de liquide fournit une fente de guidage de liquide (109) ; et
dans laquelle l'isolant avec des trous de ventilation (104) est fixé dans la cavité de bague conductrice.

2. Tête d'atomisation universelle remplaçable selon la revendication 1, **caractérisée en ce que** :
ladite buse de guidage de liquide comprend une fente de guidage de liquide, et ledit siège de buse de guidage de liquide comprend une fente de guidage de liquide, et où le dispositif de chauffage est ajusté dans la chambre d'atomisation de la base de support, ses deux extrémités étant dans la même ligne horizontale.

3. Tête d'atomisation universelle remplaçable selon la revendication 1, **caractérisée en ce que** :
ladite buse de guidage de liquide comprend deux ou trois fentes de guidage de liquide, et ledit siège de buse de guidage de liquide comprend deux ou trois fentes de guidage de liquide, où le dispositif de chauffage est situé dans la chambre d'atomisation de la base de support avec une extrémité élevée et l'autre extrémité basse.

4. Tête d'atomisation universelle remplaçable selon l'une des revendications 1 à 3, **caractérisé en ce que** :
un élément d'absorption de liquide (105) est prévu entre la base de support et la bague conductrice.

5. Atomiseur comprenant la tête d'atomisation universelle remplaçable selon la revendication 1, dans lequel il comprend en outre un ensemble d'atomisation comprenant un manchon pourvu d'un filetage extérieur, **caractérisé en ce que** :
ledit ensemble d'atomisation fournit une cavité de réception universelle pour recevoir la tête d'atomisation universelle, qui est chargée de manière amovible dans la cavité de réception, où ledit manchon extérieur fileté, relié à l'ensemble d'atomisation, est en contact avec les bagues conductrices et les conducteurs de la tête d'atomisation universelle.

6. Atomiseur selon la revendication 5, **caractérisé en ce que** :
ledit ensemble d'atomisation comprend une cartouche (200), un boîtier (201), et un premier manchon (202), où la cartouche est reliée à une extrémité du boîtier, où le premier manchon est fixé à l'autre extrémité du boîtier, où le premier manchon a un alésage étagé, dans lequel la plus petite section d'ouverture (203) de l'alésage étagé est une cavité de réception sur l'ensemble d'atomisation permettant de recevoir la tête d'atomisation universelle.

7. Atomiseur selon la revendication 6, **caractérisé en ce que** :
une saillie de retenue annulaire sur le premier manchon est définie correspondant à la paroi extérieure du segment (204) de la grande ouverture, et une gaine d'isolation emmanchée sur le premier manchon est définie correspondant à la paroi extérieure du segment de la petite ouverture.

8. Atomiseur selon la revendication 5, **caractérisé en ce que** :
ledit manchon fileté extérieur comprend un manchon (206), une électrode négative conductrice (207), une électrode positive conductrice (208) et un élément isolant (209), où une extrémité de ladite électrode négative conductrice située dans le manchon comprend un filetage extérieur, l'électrode négative conductrice comprend un trou traversant étagé, dans lequel est située l'électrode positive conductrice, où l'élément isolant est interposé dans l'électrode positive conductrice et l'électrode négative conducteur.

9. Atomiseur selon l'une des revendications 5 à 8, **caractérisé en ce que** :
ledit ensemble d'atomisation et le manchon fileté extérieur sont reliés par filetage ;
ou l'ensemble d'atomisation couvre le manchon fileté extérieur par vissage ;
ou la tige d'atomisation couvre le manchon fileté extérieur fixé par un anneau de retenue ;
ou l'ensemble d'atomisation couvre le manchon fileté extérieur pour former une connexion enfichable.

10. Cigarette électronique comprenant l'atomiseur selon la revendication 5, dans laquelle la cigarette électronique comprend en outre une tige de batterie (300), **caractérisée en ce que** : la tige de batterie est reliée par filetage au manchon fileté extérieur de l'atomiseur.
